# EUROPEAN PATENT APPLICATION

(11) **EP 4 850 699 A1**
(43) Date of publication of application: **09.12.2026**
(21) Application number: 25747855.2
(22) Date of filing: 24.01.2025
(51) Int. Cl.: A61K 47/68, A61P 35/00

(54) **USE OF ANTI-HER2 ANTIBODY-DRUG CONJUGATE FOR TREATING INTESTINAL CANCER**

(30) Priority: 29.01.2024 CN 202410126770
(71) Applicant: Chia Tai Tianqing Pharmaceutical Group Co., Ltd., Lianyungang, Jiangsu 222062 (CN)
(72) Inventor: LV, Peng, Lianyungang, Jiangsu 222062 (CN); SHEN, Chen, Lianyungang, Jiangsu 222062 (CN); TIAN, Xin, Lianyungang, Jiangsu 222062 (CN); LI, Xu, Lianyungang, Jiangsu 222062 (CN); TANG, Shaonan, Lianyungang, Jiangsu 222062 (CN); YU, Ding, Lianyungang, Jiangsu 222062 (CN); WANG, Xunqiang, Lianyungang, Jiangsu 222062 (CN); ZHANG, Xiquan, Lianyungang, Jiangsu 222062 (CN)
(74) Representative: Lorenz Seidler Gossel Part. mbB
(86) International application number: PCT/CN2025/074789
(87) International publication number: WO 2025/162287

(57) **Abstract**

An anti-HER2 antibody-drug conjugate for treating intestinal cancer in a subject. A use of the anti-HER2 antibody-drug conjugate in the preparation of a drug for treating intestinal cancer in a subject.

## Description

### TECHNICAL FIELD

The present disclosure pertains to the field of biomedicine, and particularly relates to use of an anti-HER2 antibody-drug conjugate for treating intestinal cancer.

### BACKGROUND

Human epidermal growth factor receptor 2 (HER2) belongs to the human epidermal growth factor receptor family, which includes EGFR (ErbB-1), HER2/c-neu (ErbB-2), HER3 (ErbB-3), and HER4 (ErbB-4). These receptors are all located on the cell surface and have similar structures. As a widely expressed receptor protein, HER2 exhibits abnormal gene amplification that leads to protein overexpression, which in turn causes abnormal activation of signaling pathways, serving as one of the main pathways for promoting the growth of solid tumors. HER2 can form homodimers and is also prone to forming heterodimers with other receptor members of the HER family, leading to phosphorylation of receptor tyrosine residues and initiation of various signaling pathways, including MPK, PI3K, JAK, STAT3, PKC, and the like, thereby resulting in cell proliferation and tumorigenesis.

Anti-HER2 targeted therapy is an important approach for treating intestinal cancer; therefore, there is an urgent need to explore drugs targeting HER2 to meet the huge clinical demand for intestinal cancer treatment.

### SUMMARY

### Method for Treating Intestinal Cancer

The present disclosure provides a method for treating intestinal cancer in a subject, which comprises administering to the subject the anti-HER2 antibody-drug conjugate of the present disclosure. The present disclosure further provides a method for treating intestinal cancer in a subject in third-line therapy or a later line of therapy, which comprises administering to the subject the anti-HER2 antibody-drug conjugate of the present disclosure. In some embodiments, in the method, the anti-HER2 antibody-drug conjugate is administered in a therapeutically effective amount.

The present disclosure further provides use of the anti-HER2 antibody-drug conjugate of the present disclosure for preparing a medicament for treating intestinal cancer in a subject. The present disclosure further provides use of the anti-HER2 antibody-drug conjugate of the present disclosure for preparing a medicament for treating intestinal cancer in a subject in third-line therapy or a later line of therapy. In some embodiments, the medicament comprises a therapeutically effective amount of the anti-HER2 antibody-drug conjugate.

The present disclosure further provides use of the anti-HER2 antibody-drug conjugate of the present disclosure for treating intestinal cancer in a subject.

The present disclosure further provides the anti-HER2 antibody-drug conjugate of the present disclosure for use in treating intestinal cancer in a subject. The present disclosure further provides the anti-HER2 antibody-drug conjugate of the present disclosure for use in treating intestinal cancer in a subject in third-line therapy or a later line of therapy. In some embodiments, the anti-HER2 antibody-drug conjugate is administered to the subject in a therapeutically effective amount.

In some embodiments, in the method or the use, the anti-HER2 antibody-drug conjugate is administered once every 1 week (q1w), every 2 weeks (q2w), every 3 weeks (q3w), or every 4 weeks (q4w). In one specific embodiment, in the method or the use, the anti-HER2 antibody-drug conjugate is administered once every 3 weeks. In some embodiments, in the method or the use, the anti-HER2 antibody-drug conjugate is administered at a dose of 1.5-9 mg/kg, 4.5-7.5 mg/kg, or 6-7.5 mg/kg each time. In some embodiments, in the method or the use, the anti-HER2 antibody-drug conjugate is administered at a dose of 1.5 mg/kg, 3 mg/kg, 4.5 mg/kg, 5 mg/kg, 6 mg/kg, 7.5 mg/kg, 9 mg/kg, or a range formed by any of the above values each time. In some embodiments, in the method or the use, the anti-HER2 antibody-drug conjugate is administered at a dose of 6 mg/kg or 7.5 mg/kg each time. In some embodiments, in the method or the use, the anti-HER2 antibody-drug conjugate is administered at a dose of 6 mg/kg each time. In some embodiments, in the method or the use, the anti-HER2 antibody-drug conjugate is administered at a dose of 7.5 mg/kg each time. In some embodiments, in the method or the use, the anti-HER2 antibody-drug conjugate is administered once every 1 week, every 2 weeks, every 3 weeks, or every 4 weeks at a dose of 1.5-9 mg/kg, 4.5-7.5 mg/kg, or 6-7.5 mg/kg of the anti-HER2 antibody-drug conjugate each time. In some embodiments, in the method or the use, the anti-HER2 antibody-drug conjugate is administered once every 3 weeks at a dose of 1.5 mg/kg, 3 mg/kg, 4.5 mg/kg, 5 mg/kg, 6 mg/kg, 7.5 mg/kg, or 9 mg/kg of the anti-HER2 antibody-drug conjugate each time. In some embodiments, in the method or the use, the anti-HER2 antibody-drug conjugate is administered once every 3 weeks at a dose of 6 mg/kg or 7.5 mg/kg of the anti-HER2 antibody-drug conjugate each time. In some embodiments, in the method or the use, the anti-HER2 antibody-drug conjugate is administered once every 3 weeks at a dose of 6 mg/kg of the anti-HER2 antibody-drug conjugate each time. In some embodiments, in the method or the use, the anti-HER2 antibody-drug conjugate is administered once every 3 weeks at a dose of 7.5 mg/kg of the anti-HER2 antibody-drug conjugate each time.

In some embodiments, in the method or the use, one treatment cycle consists of 1 week, 2 weeks, 3 weeks, or 4 weeks. In some embodiments, in the method or the use, one treatment cycle consists of 3 weeks. In some embodiments, in the method or the use, one treatment cycle consists of 1 week, 2 weeks, 3 weeks, or 4 weeks, and the anti-HER2 antibody-drug conjugate is administered once per treatment cycle. In some embodiments, in the method or the use, one treatment cycle consists of 3 weeks, and the anti-HER2 antibody-drug conjugate is administered once per treatment cycle. In some embodiments, in the method or the use, one treatment cycle consists of 3 weeks, and 6 mg/kg or 7.5 mg/kg of the anti-HER2 antibody-drug conjugate is administered per treatment cycle. In some embodiments, in the method or the use, one treatment cycle consists of 3 weeks, and 6 mg/kg or 7.5 mg/kg of the anti-HER2 antibody-drug conjugate is administered on day 1 per treatment cycle. In some embodiments, in the method or the use, one treatment cycle consists of 3 weeks, and 6 mg/kg of the anti-HER2 antibody-drug conjugate is administered per treatment cycle. In some embodiments, in the method or the use, one treatment cycle consists of 3 weeks, and 6 mg/kg of the anti-HER2 antibody-drug conjugate is administered on day 1 per treatment cycle. In some embodiments, in the method or the use, one treatment cycle consists of 3 weeks, and 7.5 mg/kg of the anti-HER2 antibody-drug conjugate is administered per treatment cycle. In some embodiments, in the method or the use, one treatment cycle consists of 3 weeks, and 7.5 mg/kg of the anti-HER2 antibody-drug conjugate is administered on day 1 per treatment cycle.

The anti-HER2 antibody-drug conjugate may be formulated together with one or more pharmaceutically acceptable excipients to prepare a suitable pharmaceutical composition (or referred to as a formulation). The pharmaceutical composition may be in a suitable dosage form. In some embodiments, the anti-HER2 antibody-drug conjugate is formulated into a formulation for parenteral administration. In some specific embodiments, the anti-HER2 antibody-drug conjugate is formulated into a formulation for intravenous or intramuscular administration. In some specific embodiments, the anti-HER2 antibody-drug conjugate may be formulated into an injection. In some specific embodiments, the anti-HER2 antibody-drug conjugate is formulated into a formulation for intravenous injection or infusion.

In some embodiments, in the method or the use, the anti-HER2 antibody-drug conjugate is administered by intravenous infusion.

In the method or the use, the administration regimen (e.g., administration cycle, administration time, administration dose, and dose adjustment) of the anti-HER2 antibody-drug conjugate may be adjusted according to the severity of the disease, the response to the disease, any treatment-related toxicity, and the age and health status of a patient. For example, the anti-HER2 antibody-drug conjugate may be administered with a delay of 1-56 days, such as a delay of 1 week, 2 weeks, 3 weeks, 4 weeks, 5 weeks, 6 weeks, 7 weeks, or 8 weeks. For another example, the administration dose of the anti-HER2 antibody-drug conjugate may be adjusted from 6 mg/kg to 4.5 mg/kg or 5 mg/kg, or from 7.5 mg/kg to 6 mg/kg.

In some embodiments, the intestinal cancer is unresectable, refractory, advanced, recurrent, and/or metastatic intestinal cancer. In some embodiments, the intestinal cancer is unresectable intestinal cancer. In some embodiments, the intestinal cancer is refractory intestinal cancer. In some embodiments, the intestinal cancer is advanced intestinal cancer. In some embodiments, the intestinal cancer is locally advanced intestinal cancer. In some embodiments, the intestinal cancer is recurrent and/or metastatic intestinal cancer. In some embodiments, the intestinal cancer is recurrent intestinal cancer. In some embodiments, the intestinal cancer is metastatic intestinal cancer. In some embodiments, the intestinal cancer is unresectable locally advanced intestinal cancer or intestinal cancer with distant metastasis. In some embodiments, the intestinal cancer is unresectable locally advanced intestinal cancer. In some embodiments, the intestinal cancer is intestinal cancer with distant metastasis.

In some embodiments, the intestinal cancer is HER2-expressing intestinal cancer. In some embodiments, the intestinal cancer is HER2-positive intestinal cancer. In some embodiments, the HER2-positive intestinal cancer is intestinal cancer determined to have HER2 expression of 3+ by immunohistochemistry (IHC) (e.g., IHC 3+). In some embodiments, the HER2-positive intestinal cancer is intestinal cancer determined to have HER2 expression of 2+ by IHC and determined to be positive for HER2 expression by ISH (e.g., IHC 2+ and ISH+).

In some embodiments, the intestinal cancer is HER2-positive unresectable intestinal cancer. In some embodiments, the intestinal cancer is HER2-positive advanced intestinal cancer. In some embodiments, the intestinal cancer is HER2-positive locally advanced intestinal cancer. In some embodiments, the intestinal cancer is advanced intestinal cancer determined to have HER2 expression of 3+ by IHC. In some embodiments, the intestinal cancer is locally advanced intestinal cancer determined to have HER2 expression of 3+ by IHC. In some embodiments, the intestinal cancer is HER2-positive metastatic intestinal cancer. In some embodiments, the intestinal cancer is metastatic intestinal cancer determined to have HER2 expression of 3+ by IHC. In some embodiments, the intestinal cancer is HER2-positive unresectable locally advanced intestinal cancer or intestinal cancer with distant metastasis. In some embodiments, the intestinal cancer is unresectable locally advanced intestinal cancer or intestinal cancer with distant metastasis determined to have HER2 expression of 3+ by IHC. In some embodiments, the intestinal cancer is HER2-positive unresectable locally advanced intestinal cancer. In some embodiments, the intestinal cancer is unresectable locally advanced intestinal cancer determined to have HER2 expression of 3+ by IHC. In some embodiments, the intestinal cancer is HER2-positive intestinal cancer with distant metastasis. In some embodiments, the intestinal cancer is intestinal cancer with distant metastasis determined to have HER2 expression of 3+ by IHC.

In some embodiments, the subject with intestinal cancer has previously received treatment for treating intestinal cancer (e.g., treatment failure or intolerance). In some embodiments, the subject with intestinal cancer has previously received at least second-line therapy for treating intestinal cancer (e.g., treatment failure or intolerance). In some embodiments, the subject with intestinal cancer has previously received at least third-line therapy for treating intestinal cancer (e.g., treatment failure or intolerance).

In some embodiments, the subject is a subject with intestinal cancer who has failed prior treatment with an HER2-targeted drug. In some embodiments, the subject is a subject with intestinal cancer who has failed prior treatment with a VEGFR-targeted drug. In some embodiments, the subject is a subject with HER2-positive intestinal cancer who has failed prior treatment with an HER2-targeted drug. In some embodiments, the subject is a subject with HER2-positive intestinal cancer who has failed prior treatment with a VEGFR-targeted drug. In some embodiments, the subject is a subject with HER2-positive advanced intestinal cancer who has failed prior treatment with an HER2-targeted drug. In some embodiments, the subject is a subject with HER2-positive advanced intestinal cancer who has failed prior treatment with a VEGFR-targeted drug. In some embodiments, the subject is a subject with HER2-positive locally advanced intestinal cancer who has failed prior treatment with an HER2-targeted drug. In some embodiments, the subject is a subject with HER2-positive locally advanced intestinal cancer who has failed prior treatment with a VEGFR-targeted drug. In some embodiments, the subject is a subject with HER2-positive unresectable locally advanced intestinal cancer who has failed prior treatment with an HER2-targeted drug. In some embodiments, the subject is a subject with HER2-positive unresectable locally advanced intestinal cancer who has failed prior treatment with a VEGFR-targeted drug. In some embodiments, the subject is a subject with HER2-positive intestinal cancer with distant metastasis who has failed prior treatment with an HER2-targeted drug. In some embodiments, the subject is a subject with HER2-positive intestinal cancer with distant metastasis who has failed prior treatment with a VEGFR-targeted drug. In some embodiments, the subject is a subject with unresectable locally advanced intestinal cancer determined to have HER2 expression of 3+ by IHC who has failed prior treatment with an HER2-targeted drug. In some embodiments, the subject is a subject with unresectable locally advanced intestinal cancer determined to have HER2 expression of 3+ by IHC who has failed prior treatment with a VEGFR-targeted drug. In some embodiments, the subject is a subject with intestinal cancer with distant metastasis determined to have HER2 expression of 3+ by IHC who has failed prior treatment with an HER2-targeted drug. In some embodiments, the subject is a subject with intestinal cancer with distant metastasis determined to have HER2 expression of 3+ by IHC who has failed prior treatment with a VEGFR-targeted drug. In some embodiments, the subject is a subject with unresectable locally advanced colorectal cancer determined to have HER2 expression of 3+ by IHC who has failed prior treatment with an HER2-targeted drug. In some embodiments, the subject is a subject with unresectable locally advanced colorectal cancer determined to have HER2 expression of 3+ by IHC who has failed prior treatment with a VEGFR-targeted drug. In some embodiments, the subject is a subject with colorectal cancer with distant metastasis determined to have HER2 expression of 3+ by IHC who has failed prior treatment with an HER2-targeted drug. In some embodiments, the subject is a subject with colorectal cancer with distant metastasis determined to have HER2 expression of 3+ by IHC who has failed prior treatment with a VEGFR-targeted drug.

In some embodiments, the subject is a subject with intestinal cancer who has failed prior treatment with one or more of anti-HER2 ADCs (including any anti-HER2 ADC currently marketed), trastuzumab, pertuzumab, pyrotinib, lapatinib, regorafenib, fruquintinib, bevacizumab, and cetuximab. In some embodiments, the subject is a subject with HER2-positive intestinal cancer who has failed prior treatment with one or more of anti-HER2 ADCs, trastuzumab, pertuzumab, pyrotinib, lapatinib, regorafenib, fruquintinib, bevacizumab, and cetuximab. In some embodiments, the subject is a subject with HER2-positive advanced intestinal cancer who has failed prior treatment with one or more of anti-HER2 ADCs, trastuzumab, pertuzumab, pyrotinib, lapatinib, regorafenib, fruquintinib, bevacizumab, and cetuximab. In some embodiments, the subject is a subject with HER2-positive locally advanced intestinal cancer who has failed prior treatment with one or more of anti-HER2 ADCs, trastuzumab, pertuzumab, pyrotinib, lapatinib, regorafenib, fruquintinib, bevacizumab, and cetuximab. In some embodiments, the subject is a subject with HER2-positive unresectable locally advanced intestinal cancer who has failed prior treatment with one or more of anti-HER2 ADCs, trastuzumab, pertuzumab, pyrotinib, lapatinib, regorafenib, fruquintinib, bevacizumab, and cetuximab. In some embodiments, the subject is a subject with HER2-positive intestinal cancer with distant metastasis who has failed prior treatment with one or more of anti-HER2 ADCs, trastuzumab, pertuzumab, pyrotinib, lapatinib, regorafenib, fruquintinib, bevacizumab, and cetuximab. In some embodiments, the subject is a subject with unresectable locally advanced intestinal cancer determined to have HER2 expression of 3+ by IHC who has failed prior treatment with one or more of anti-HER2 ADCs, trastuzumab, pertuzumab, pyrotinib, lapatinib, regorafenib, fruquintinib, bevacizumab, and cetuximab. In some embodiments, the subject is a subject with intestinal cancer with distant metastasis determined to have HER2 expression of 3+ by IHC who has failed prior treatment with one or more of anti-HER2 ADCs, trastuzumab, pertuzumab, pyrotinib, lapatinib, regorafenib, fruquintinib, bevacizumab, and cetuximab. In some embodiments, the subject is a subject with unresectable locally advanced colorectal cancer determined to have HER2 expression of 3+ by IHC who has failed prior treatment with one or more of anti-HER2 ADCs, trastuzumab, pertuzumab, pyrotinib, lapatinib, regorafenib, fruquintinib, bevacizumab, and cetuximab. In some embodiments, the subject is a subject with colorectal cancer with distant metastasis determined to have HER2 expression of 3+ by IHC who has failed prior treatment with one or more of anti-HER2 ADCs, trastuzumab, pertuzumab, pyrotinib, lapatinib, regorafenib, fruquintinib, bevacizumab, and cetuximab.

In some embodiments, the subject with intestinal cancer has previously received standard therapy for treating intestinal cancer (e.g., treatment failure or intolerance). In some embodiments, the subject with intestinal cancer has previously received standard therapy for treating intestinal cancer, and has experienced disease progression or intolerance during treatment or after the end of treatment. In some embodiments, the subject with intestinal cancer has previously received standard therapy for treating intestinal cancer, and has experienced disease progression or intolerance during treatment or within 3 months (e.g., 1, 2, or 3 months) after the end of treatment. In some embodiments, the subject with intestinal cancer has previously received chemotherapy for treating intestinal cancer (e.g., treatment failure or intolerance). In some embodiments, the subject with intestinal cancer has previously received standard chemotherapy for treating intestinal cancer (e.g., treatment failure or intolerance). In some embodiments, the standard therapy comprises administering to the subject a drug including but not limited to, fluorouracil or a derivative thereof, oxaliplatin, and/or irinotecan. In some embodiments, the standard chemotherapy comprises administering to the subject a drug including but not limited to, fluorouracil or a derivative thereof, oxaliplatin, and/or irinotecan. In some embodiments, the subject with intestinal cancer has previously received treatment with fluorouracil or a derivative thereof, oxaliplatin, and/or irinotecan for treating intestinal cancer (e.g., treatment failure or intolerance). In some embodiments, the subject is a subject with intestinal cancer who has failed prior treatment with a 5-fluorouracil drug, oxaliplatin, and/or irinotecan. In some embodiments, the subject is a subject with HER2-positive intestinal cancer who has failed prior treatment with a 5-fluorouracil drug, oxaliplatin, and/or irinotecan. In some embodiments, the subject is a subject with intestinal cancer determined to have HER2 expression of 3+ by IHC who has failed prior treatment with a 5-fluorouracil drug, oxaliplatin, and/or irinotecan. In some embodiments, the subject is a subject with advanced intestinal cancer determined to have HER2 expression of 3+ by IHC who has failed prior treatment with a 5-fluorouracil drug, oxaliplatin, and/or irinotecan. In some embodiments, the subject is a subject with advanced colorectal cancer with HER2 expression of 3+ who has failed prior treatment with a 5-fluorouracil drug, oxaliplatin, and/or irinotecan. In some embodiments, the subject with intestinal cancer has previously received adjuvant therapy for treating intestinal cancer (e.g., treatment failure or intolerance). In some embodiments, the subject with intestinal cancer has previously received adjuvant therapy for treating intestinal cancer, and has experienced disease progression during treatment or after the end of treatment. In some embodiments, the subject with intestinal cancer has previously received adjuvant therapy for treating intestinal cancer (e.g., treatment failure or intolerance), the adjuvant therapy comprising administering oxaliplatin to the subject. In some embodiments, the subject with intestinal cancer has previously received adjuvant therapy for treating intestinal cancer, the adjuvant therapy comprising administering oxaliplatin to the subject, and has experienced disease progression during treatment or within 6 months (e.g., 1, 2, 3, 4, 5, or 6 months) after the end of treatment. In some embodiments, the intolerance is defined as grade 4 hematological toxicity. The hematological toxicity includes, but is not limited to, decreased neutrophil count, decreased leukocyte count, anemia, decreased lymphocyte count, and decreased platelet count. In some embodiments, the intolerance is defined as a reduction in platelet count of grade 3 or higher. In some embodiments, the intolerance is defined as grade 3 or higher non-hematological toxicity.

In some embodiments, the subject with intestinal cancer has previously received local radiation therapy for treating intestinal cancer. In some embodiments, the subject with intestinal cancer has previously received local radiation therapy for treating intestinal cancer, the local radiation therapy is given more than 2 weeks prior to the first administration of the anti-HER2 antibody-drug conjugate, and a lesion targeted by the anti-HER2 antibody-drug conjugate is not within a local radiation therapy area. In some embodiments, the subject with intestinal cancer has previously received local radiation therapy for treating intestinal cancer, the local radiation therapy being given more than 2 weeks prior to the first administration of the anti-HER2 antibody-drug conjugate, and has experienced disease progression. In some embodiments, the local radiation therapy is palliative radiation therapy or brain radiation therapy.

In some embodiments, the intestinal cancer is sigmoid colon cancer, colon cancer, rectal cancer, or colorectal cancer. In some embodiments, the intestinal cancer is protruding type intestinal cancer, ulcer type intestinal cancer, or invasive type intestinal cancer.

In some embodiments, the intestinal cancer is stage I, stage II, stage III, or stage IV intestinal cancer according to TNM staging criteria.

### Anti-HER2 Antibody-Drug Conjugate

The anti-HER2 antibody-drug conjugate used in the present disclosure is formed by linking a drug-linker having a structure represented by formula Ia below to an HER2-targeting antigen-binding construct: 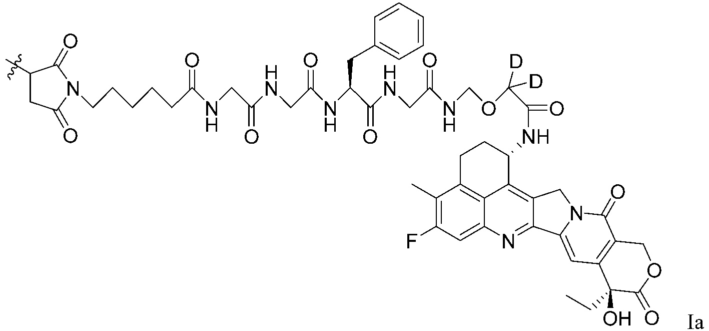 wherein,
position 3 of -(succinimid-3-yl-N)- in formula Ia (i.e., the position linked with " 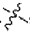 ") is linked to the HER2-targeting antigen-binding construct;
the HER2-targeting antigen-binding construct comprises a first antigen-binding fragment and a second antigen-binding fragment, wherein the first antigen-binding fragment comprises: a heavy chain CDR1 (HCDR1) comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, a light chain CDR1 (LCDR1) comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6, and the second antigen-binding fragment comprises: an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.

In some embodiments, position 3 of -(succinimid-3-yl-N)- is linked to the HER2-targeting antigen-binding construct via a thioether bond.

In some embodiments, in the anti-HER2 antibody-drug conjugate, the average number of linked drug-linkers per HER2-targeting antigen-binding construct is 2 to 8. In some embodiments, in the anti-HER2 antibody-drug conjugate, the average number of linked drug-linkers per HER2-targeting antigen-binding construct is 4 to 7. In some embodiments, in the anti-HER2 antibody-drug conjugate, the average number of linked drug-linkers per HER2-targeting antigen-binding construct is 5 to 6. In some embodiments, in the anti-HER2 antibody-drug conjugate, the average number of linked drug-linkers per HER2-targeting antigen-binding construct is 5.5 to 6. In some embodiments, in the anti-HER2 antibody-drug conjugate, the average number of linked drug-linkers per HER2-targeting antigen-binding construct is 5.8 to 6.

**Table 1. CDR sequences of exemplary HER2-targeting antigen-binding construct**

| | | | |
|---|---|---|---|
| First antigen-binding fragment | HCDR1 | GFNIEDTYIH | SEQ ID NO: 1 |
| | HCDR2 | RIYPTNGYTRYADSVKG | SEQ ID NO: 2 |
| | HCDR3 | WGGDGFYAMDYW | SEQ ID NO: 3 |
| | LCDR1 | CRASQDVNTAVAW | SEQ ID NO: 4 |
| | LCDR2 | SASYLYS | SEQ ID NO: 5 |
| | LCDR3 | QQHYTTPPT | SEQ ID NO: 6 |
| Second antigen-binding fragment | HCDR1 | GFTFTDYTMD | SEQ ID NO: 9 |
| | HCDR2 | DVNPNSGGSIYNQRFKG | SEQ ID NO: 10 |
| | HCDR3 | NLGPSFYFDY | SEQ ID NO: 11 |
| | LCDR1 | KASQDVSIGVA | SEQ ID NO: 12 |
| | LCDR2 | SASYRYT | SEQ ID NO: 13 |
| | LCDR3 | QQYYIYPYT | SEQ ID NO: 14 |

It should be understood by those skilled in the art that, unless otherwise specified, the term "CDR" or "complementarity determining region" of a given antigen-binding fragment or a region thereof (e.g., a variable region) should be understood to encompass complementarity determining regions defined by any one of the known schemes. Although the CDRs claimed in the present disclosure are based on the sequences shown in Table 1 (one definition scheme), amino acid sequences corresponding to other CDR definition schemes (one of or a combination of several of the definition schemes well known in the art, such as AbM, CCG, Kabat, Chothia, IMGT, or Contact) should also fall within the protection scope of the present disclosure.

In some embodiments, the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8. In some embodiments, the first antigen-binding fragment comprises a heavy chain variable region having the amino acid sequence set forth in SEQ ID NO: 7 and a light chain variable region having the amino acid sequence set forth in SEQ ID NO: 8. In some specific embodiments, the amino acid sequence of the heavy chain variable region of the first antigen-binding fragment is set forth in SEQ ID NO: 7, and the amino acid sequence of the light chain variable region of the first antigen-binding fragment is set forth in SEQ ID NO: 8.

In some embodiments, the first antigen-binding fragment comprises a heavy chain variable region and a light chain variable region, wherein the heavy chain variable region comprises an HCDR1 having the amino acid sequence set forth in SEQ ID NO: 1, an HCDR2 having the amino acid sequence set forth in SEQ ID NO: 2, and an HCDR3 having the amino acid sequence set forth in SEQ ID NO: 3, and the light chain variable region comprises an LCDR1 having the amino acid sequence set forth in SEQ ID NO: 4, an LCDR2 having the amino acid sequence set forth in SEQ ID NO: 5, and an LCDR3 having the amino acid sequence set forth in SEQ ID NO: 6; and, the heavy chain variable region comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to SEQ ID NO: 7, and the light chain variable region comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to SEQ ID NO: 8.

EVQLVESGGGLVQPGGSLRLSCAASGFNIEDTYIHWVRQAPGKGLEWVARIYPTNGYTRYADSVKGRFTI
SADTSKNTAYLQMNSLRAEDTAVYYCSRWGGDGFYAMDYWGQGTLVTVSS (SEQ ID NO: 7);

DIQMTQSPSSLSASVGDRVTITCRASQDVNTAVAWYQQKPGKAPKLLIYSASYLYSGVPSRFSGSRSGTDF
TLTISSLQPEDFATYYCQQHYTTPPTFGQGTKVEIK (SEQ ID NO: 8).

In some embodiments, compared with the amino acid sequence set forth in SEQ ID NO: 7 or SEQ ID NO: 8, the differing amino acids in an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity thereto are located in FR regions. In some embodiments, the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16. In some embodiments, the second antigen-binding fragment comprises a heavy chain variable region having the amino acid sequence set forth in SEQ ID NO: 15 and a light chain variable region having the amino acid sequence set forth in SEQ ID NO: 16. In some specific embodiments, the amino acid sequence of the heavy chain variable region of the second antigen-binding fragment is set forth in SEQ ID NO: 15, and the amino acid sequence of the light chain variable region of the second antigen-binding fragment is set forth in SEQ ID NO: 16.

In some embodiments, the second antigen-binding fragment comprises a heavy chain variable region and a light chain variable region, wherein the heavy chain variable region comprises an HCDR1 having the amino acid sequence set forth in SEQ ID NO: 9, an HCDR2 having the amino acid sequence set forth in SEQ ID NO: 10, and an HCDR3 having the amino acid sequence set forth in SEQ ID NO: 11, and the light chain variable region comprises an LCDR1 having the amino acid sequence set forth in SEQ ID NO: 12, an LCDR2 having the amino acid sequence set forth in SEQ ID NO: 13, and an LCDR3 having the amino acid sequence set forth in SEQ ID NO: 14; and, the heavy chain variable region comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to SEQ ID NO: 15, and the light chain variable region comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to SEQ ID NO: 16.


 (SEQ ID NO: 15);


 (SEQ ID NO: 16).

In some embodiments, compared with the amino acid sequence set forth in SEQ ID NO: 15 or SEQ ID NO: 16, the differing amino acids in an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity thereto are located in the FR regions.

In some embodiments, the HER2-targeting antigen-binding construct may further comprise a constant region of an immunoglobulin, or a fragment, an analog, a variant, or a derivative of the constant region. In some embodiments, the constant region comprises a heavy chain constant region and a light chain constant region. In some embodiments, the heavy chain constant region is derived from a human immunoglobulin heavy chain, for example, a heavy chain of IgG1, IgG2, IgG3, and IgG4, or other types of immunoglobulins, preferably a heavy chain of IgG1. In some embodiments, the light chain constant region is derived from a human immunoglobulin light chain, for example, a κ light chain or a λ light chain of a human immunoglobulin. In some embodiments, the constant region may comprise any modification described in the text, for example, insertion, deletion, substitution, or chemical modification of amino acids. In some embodiments, the C-terminal lysine of the heavy chain constant region may be present or deleted, and the deletion of the C-terminal lysine of the heavy chain constant region generally occurs during recombinant expression. In some embodiments, the constant region comprises a mutation that alters the effector function. In some embodiments, any amino acid residue of the constant region may be substituted with an amino acid residue of any allotype.

In some embodiments, the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19. In some embodiments, the HER2-targeting antigen-binding construct comprises a first polypeptide chain having the amino acid sequence set forth in SEQ ID NO: 17, a second polypeptide chain having the amino acid sequence set forth in SEQ ID NO: 18, and a third polypeptide chain having the amino acid sequence set forth in SEQ ID NO: 19. In some specific embodiments, the HER2-targeting antigen-binding construct consists of three polypeptide chains, wherein the amino acid sequence of the first polypeptide chain is set forth in SEQ ID NO: 17, the amino acid sequence of the second polypeptide chain is set forth in SEQ ID NO: 18, and the amino acid sequence of the third polypeptide chain is set forth in SEQ ID NO: 19.

In some embodiments, the HER2-targeting antigen-binding construct comprises a first polypeptide chain, a second polypeptide chain, and a third polypeptide chain, wherein the first polypeptide chain comprises an HCDR1 having the amino acid sequence set forth in SEQ ID NO: 1, an HCDR2 having the amino acid sequence set forth in SEQ ID NO: 2, an HCDR3 having the amino acid sequence set forth in SEQ ID NO: 3, an LCDR1 having the amino acid sequence set forth in SEQ ID NO: 4, an LCDR2 having the amino acid sequence set forth in SEQ ID NO: 5, and an LCDR3 having the amino acid sequence set forth in SEQ ID NO: 6, the second polypeptide chain comprises an HCDR1 having the amino acid sequence set forth in SEQ ID NO: 9, an HCDR2 having the amino acid sequence set forth in SEQ ID NO: 10, and an HCDR3 having the amino acid sequence set forth in SEQ ID NO: 11, and the third polypeptide chain comprises an LCDR1 having the amino acid sequence set forth in SEQ ID NO: 12, an LCDR2 having the amino acid sequence set forth in SEQ ID NO: 13, and an LCDR3 having the amino acid sequence set forth in SEQ ID NO: 14; and, the first polypeptide chain comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17, the second polypeptide chain comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18, and the third polypeptide chain comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19.

EVQLVESGGGLVQPGGSLRLSCAASGFNIEDTYIHWVRQAPGKGLEWVARIYPTNGYTRYADSVKGRFTI
SADTSKNTAYLQMNSLRAEDTAVYYCSRWGGDGFYAMDYWGQGTLVTVSSGGGGSGGGGSGGGGSGG
GGSDIQMTQSPSSLSASVGDRVTITCRASQDVNTAVAWYQQKPGKAPKLLIYSASYLYSGVPSRFSGSRSG
TDFTLTISSLQPEDFATYYCQQHYTTPPTFGQGTKVEIKGEPKSSDKTHTCPPCPAPELLGGPSVFLFPPKPK
DTLMISRTPEVTCVVVDVSHEDPEVKFNWYVDGVEVHNAKTKPREEQYNSTYRVVSVLTVLHQDWLNG
KEYKCKVSNKALPAPIEKTISKAKGQPREPQVYTLPPCREEMTKNQVSLWCLVKGFYPSDIAVEWESNGQ
PENNYKTTPPVLDSDGSFFLYSKLTVDKSRWQQGNVFSCSVMHEALHNHYTQKSLSLSPGK (SEQ ID NO:
17);

EVQLVESGGGLVQPGGSLRLSCAASGFTFTDYTMDWVRQAPGKGLEWVADVNPNSGGSIYNQRFKGRF
TLSVDRSKNTLYLQMNSLRAEDTAVYYCARNLGPSFYFDYWGQGTLVTVSSASTKGPSVFPLAPSSKSTS
GGTAALGCLVKDYFPEPVTVSWNSGALTSGVHTFPAVLQSSGLYSLSSVVTVPSSSLGTQTYICNVNHKPS
NTKVDKKVEPKSCDKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEKTISKAKGQPREP
QVCTLPPSREEMTKNQVSLSCAVKGFYPSDIAVEWESNGQPENNYKTTPPVLDSDGSFFLVSKLTVDKSR
WQQGNVFSCSVMHEALHNRFTQKSLSLSPGK (SEQ ID NO: 18);

DIQMTQSPSSLSASVGDRVTITCKASQDVSIGVAWYQQKPGKAPKLLIYSASYRYTGVPSRFSGSGSGTDF
TLTISSLQPEDFATYYCQQYYIYPYTFGQGTKVEIKRTVAAPSVFIFPPSDEQLKSGTASVVCLLNNFYPREA
KVQWKVDNALQSGNSQESVTEQDSKDSTYSLSSTLTLSKADYEKHKVYACEVTHQGLSSPVTKSFNRGE
C (SEQ ID NO: 19).

In some embodiments, the C-terminal lysine of the amino acid sequence set forth in SEQ ID NO: 17 is deleted, as set forth in SEQ ID NO: 20. In some embodiments, the C-terminal lysine of the amino acid sequence set forth in SEQ ID NO: 18 is deleted, as set forth in SEQ ID NO: 21. In some embodiments, the C-terminal lysine of each of the first polypeptide chain and the second polypeptide chain is deleted, the amino acid sequence of the first polypeptide chain is set forth in SEQ ID NO: 20, and the amino acid sequence of the second polypeptide chain is set forth in SEQ ID NO: 21.

EVQLVESGGGLVQPGGSLRLSCAASGFNIEDTYIHWVRQAPGKGLEWVARIYPTNGYTRYADSVKGRFTI
SADTSKNTAYLQMNSLRAEDTAVYYCSRWGGDGFYAMDYWGQGTLVTVSSGGGGSGGGGSGGGGSGG
GGSDIQMTQSPSSLSASVGDRVTITCRASQDVNTAVAWYQQKPGKAPKLLIYSASYLYSGVPSRFSGSRSG
TDFTLTISSLQPEDFATYYCQQHYTTPPTFGQGTKVEIKGEPKSSDKTHTCPPCPAPELLGGPSVFLFPPKPK
DTLMISRTPEVTCVVVDVSHEDPEVKFNWYVDGVEVHNAKTKPREEQYNSTYRVVSVLTVLHQDWLNG

KEYKCKVSNKALPAPIEKTISKAKGQPREPQVYTLPPCREEMTKNQVSLWCLVKGFYPSDIAVEWESNGQ
PENNYKTTPPVLDSDGSFFLYSKLTVDKSRWQQGNVFSCSVMHEALHNHYTQKSLSLSPG (SEQ ID NO:
20);

EVQLVESGGGLVQPGGSLRLSCAASGFTFTDYTMDWVRQAPGKGLEWVADVNPNSGGSIYNQRFKGRF
TLSVDRSKNTLYLQMNSLRAEDTAVYYCARNLGPSFYFDYWGQGTLVTVSSASTKGPSVFPLAPSSKSTS
GGTAALGCLVKDYFPEPVTVSWNSGALTSGVHTFPAVLQSSGLYSLSSVVTVPSSSLGTQTYICNVNHKPS
NTKVDKKVEPKSCDKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEKTISKAKGQPREP
QVCTLPPSREEMTKNQVSLSCAVKGFYPSDIAVEWESNGQPENNYKTTPPVLDSDGSFFLVSKLTVDKSR
WQQGNVFSCSVMHEALHNRFTQKSLSLSPG (SEQ ID NO: 21).

In some embodiments, compared with the amino acid sequence set forth in SEQ ID NO: 17, 18, or 19, the differing amino acids in an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity thereto are located in FR regions or constant regions.

In some other embodiments, the HER2-targeting antigen-binding construct is selected from Expi Her2-1, Expi Her2-3, Expi Her2-4, Expi Her2-5, 23C2 Her2-1, 23C2 Her2-3, 23C2 Her2-4, and 23C2 Her2-5 (see WO2021219046 or CN115279791A). In some other embodiments, the HER2-targeting antigen-binding construct is selected from zanidatamab (ZW25), KN026, MBS301, KM257, and BCD-147.

The anti-HER2 antibody-drug conjugate used in the present disclosure may also be represented by the structure represented by formula II below: 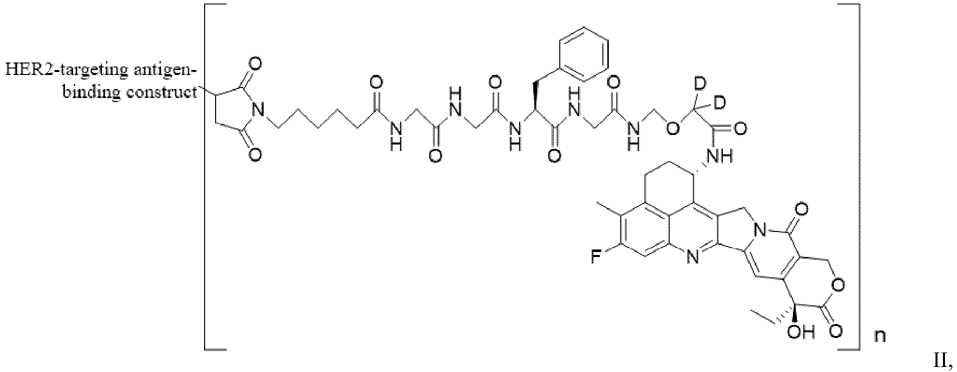 the HER2-targeting antigen-binding construct is as described above. In some embodiments, the drug-linker is linked to the HER2-targeting antigen-binding construct via a thioether bond (position 3 of -(succinimid-3-yl-N)-). n has the same meaning as DAR and represents the average number of linked cytotoxic drugs per HER2-targeting antigen-binding construct. In some embodiments, n is 2 to 8. In some embodiments, n is 4 to 7. In some embodiments, n is 5 to 6. In some embodiments, n is 5.5 to 6. In some embodiments, n is 5.8 to 6.

The antibody-drug conjugate preferably used in the present disclosure is formed by linking a drug-linker having a structure represented by formula Ia below to an HER2-targeting antigen-binding construct: 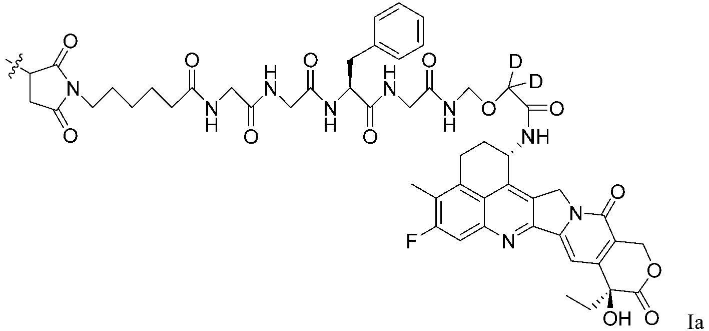 wherein,
position 3 of -(succinimid-3-yl-N)- in formula Ia is linked to the HER2-targeting antigen-binding construct;
the HER2-targeting antigen-binding construct consists of three polypeptide chains, wherein the amino acid sequence of the first polypeptide chain is set forth in SEQ ID NO: 17 or a variant thereof with a deletion of lysine at the C-terminus, the amino acid sequence of the second polypeptide chainis set forth in SEQ ID NO: 18 or a variant thereof with a deletion of lysine at the C-terminus, and the amino acid sequence of the third polypeptide chain is set forth in SEQ ID NO: 19 (for example, the amino acid sequence of the first polypeptide chain is set forth in SEQ ID NO: 17, the amino acid sequence of the second polypeptide chain is set forth in SEQ ID NO: 18, and the amino acid sequence of the third polypeptide chain is set forth in SEQ ID NO: 19; or the amino acid sequence of the first polypeptide chain is set forth in SEQ ID NO: 20, the amino acid sequence of the second polypeptide chain is set forth in SEQ ID NO: 21, and the amino acid sequence of the third polypeptide chain is set forth in SEQ ID NO: 19; or the amino acid sequence of the first polypeptide chain is set forth in SEQ ID NO: 20, the amino acid sequence of the second polypeptide chain is set forth in SEQ ID NO: 18, and the amino acid sequence of the third polypeptide chain is set forth in SEQ ID NO: 19; or the amino acid sequence of the first polypeptide chain is set forth in SEQ ID NO: 17, the amino acid sequence of the second polypeptide chain is set forth in SEQ ID NO: 21, and the amino acid sequence of the third polypeptide chain is set forth in SEQ ID NO: 19), and
the average number of linked drug-linkers for one HER2-targeting antigen-binding construct is 5 to 6.

In some embodiments, position 3 of -(succinimid-3-yl-N)- in formula Ia is linked to the HER2-targeting antigen-binding construct via a thioether bond.

The antibody-drug conjugate preferably used in the present disclosure may also be represented by the structure represented by formula II below: 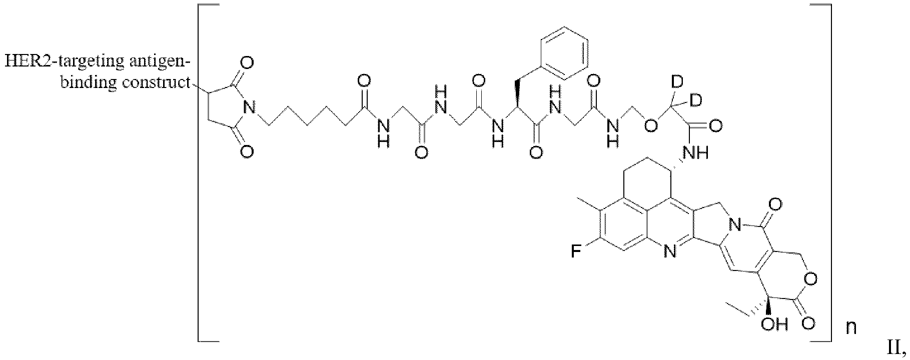
wherein n is 5 to 6;
the HER2-targeting antigen-binding construct consists of three polypeptide chains, wherein the amino acid sequence of the first polypeptide chain is set forth in SEQ ID NO: 17 or a variant thereof with a deletion of lysine at the C-terminus, the amino acid sequence of the second polypeptide chain is set forth in SEQ ID NO: 18 or a variant thereof with a deletion of lysine at the C-terminus, and the amino acid sequence of the third polypeptide chain is set forth in SEQ ID NO: 19 (for example, the amino acid sequence of the first polypeptide chain is set forth in SEQ ID NO: 17, the amino acid sequence of the second polypeptide chain is set forth in SEQ ID NO: 18, and the amino acid sequence of the third polypeptide chain is set forth in SEQ ID NO: 19; or the amino acid sequence of the first polypeptide chain is set forth in SEQ ID NO: 20, the amino acid sequence of the second polypeptide chain is set forth in SEQ ID NO: 21, and the amino acid sequence of the third polypeptide chain is set forth in SEQ ID NO: 19; or the amino acid sequence of the first polypeptide chain is set forth in SEQ ID NO: 20, the amino acid sequence of the second polypeptide chain is set forth in SEQ ID NO: 18, and the amino acid sequence of the third polypeptide chain is set forth in SEQ ID NO: 19; or the amino acid sequence of the first polypeptide chain is set forth in SEQ ID NO: 17, the amino acid sequence of the second polypeptide chain is set forth in SEQ ID NO: 21, and the amino acid sequence of the third polypeptide chain is set forth in SEQ ID NO: 19). In some embodiments, the drug-linker is linked to the HER2-targeting antigen-binding construct via a thioether bond.

In some other specific embodiments, the anti-HER2 antibody-drug conjugate of the present disclosure is selected from Kadcyla^{®}, Enhertu^{®}, Aidixi^{®}, MRG002, ARX788, A166, SHR-A1811, BB-1701, SYD985, FS-1502, and BAT8001.

The anti-HER2 antibody-drug conjugate described in the present disclosure also includes isomers or pharmaceutically acceptable salts of the anti-HER2 antibody-drug conjugate, or solvates of the anti-HER2 antibody-drug conjugate, isomers thereof, or pharmaceutically acceptable salts thereof.

### Technical Effects

Administration of the anti-HER2 antibody-drug conjugate of the present disclosure achieves one or more of the following effects:
(1) causing benefit to subjects with intestinal cancer, wherein the intestinal cancer is preferably unresectable locally advanced intestinal cancer or metastatic intestinal cancer (e.g., intestinal cancer with distant metastasis);
(2) exhibiting good safety;
(3) being well-tolerated in subjects.

### Definitions and Description

Unless otherwise stated, the following terms used in the present disclosure shall have the following meanings. A certain term, unless otherwise specifically defined, should not be considered uncertain or unclear, but interpreted according to its common meaning in the art. When referring to a trade name in the present disclosure, it is intended to refer to its corresponding commercial product or its active ingredient.

As used herein, the structure of "-(succinimid-3-yl-N)-" is as the formula below:

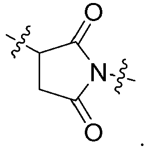

Unless otherwise stated, the absolute configuration of a stereogenic center is represented by a wedged solid bond ( 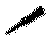 ) and a wedged dashed bond ( 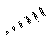 ).

Unless otherwise specified, when a certain group has a linkable site, the linkage between the site and another group may be represented by a wavy line ( 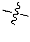 ).

The term "antigen-binding construct" refers to any agent capable of binding to an antigen, such as a polypeptide or a polypeptide complex. In some aspects, the antigen-binding construct is a polypeptide that specifically binds to a target antigen. The antigen-binding construct may be a monomer, a dimer, a polymer, a protein, a peptide, a protein or peptide complex, an antibody or an antigen-binding fragment thereof, and the like. The antigen-binding construct may be a monospecific, bispecific, or multispecific polypeptide construct. In some aspects, the antigen-binding construct may include, for example, one or more antigen-binding fragments (e.g., Fab or scFv) linked to one or more Fc.

The "antigen-binding fragment" of an antibody refers to one or more fragments of the antibody that retain the functionality of specifically binding to an antigen (e.g., HER2 protein). It has been demonstrated that the antigen-binding functionality of an antibody can be implemented by fragments of a full-length antibody. Examples encompassed within the term "antigen-binding fragment" of an antibody include: (i) a Fab fragment: a monovalent fragment consisting of VL, VH, CL, and CH1 domains; (ii) a F(ab')₂ fragment, a bivalent fragment comprising two Fab fragments linked by a disulfide bridge at the hinge region; (iii) an Fd fragment consisting of VH and CH1 domains; (iv) an Fv fragment consisting of VL and VH domains of a single arm of the antibody; (v) a dAb fragment consisting of VH domains (see Ward et al., Nature. 341:544-546 (1989)); and (vi) a nanobody, an antibody comprising a single variable domain and two constant domains. Furthermore, although the two domains VL and VH of the Fv fragment are encoded by different genes, the VH and VL can be joined via a linker by recombinant methods into a single protein chain in which the VL and VH are paired to form a monovalent molecule referred to as a single-chain Fv (scFv) (see, Bird et al., Science. 242:423-426 (1988); Huston et al., Proc. Natl. Acad. Sci. 85:5879-5883 (1988)). These single-chain antibodies are also encompassed within the term "antigen-binding fragment". These antibody fragments can be obtained using conventional techniques known to those skilled in the art, and the fragments can be subjected to functional screening using the same method as full-length antibodies.

The term "identity" is also referred to as consistency. The "percent identity (%)" of an amino acid sequence refers to the percentage of amino acid residues in a sequence to be aligned that are identical to those of a specific amino acid sequence as set forth herein when the sequence to be aligned is aligned with the specific amino acid sequence as set forth herein, with gaps introduced, if necessary, to achieve the maximum percent sequence identity and without considering any conservative replacements as part of the sequence identity. The alignment of amino acid sequences for identity can be performed in a variety of ways within the skill in the art, such as BLAST, BLAST-2, ALIGN, or Megalign (DNASTAR) software. Those skilled in the art can determine suitable parameters for aligning sequences, including any algorithms required to acquire the maximum alignment for the full length of sequences being compared.

The term "treating" refers to administering the compound described in the present disclosure to prevent, ameliorate, or eliminate a disease or one or more symptoms associated with the disease, and includes, but is not limited to:
(i) preventing the occurrence of a disease or disease state in a mammal, particularly when such a mammal is predisposed to the disease state but has not yet been diagnosed as having it;
(ii) inhibiting a disease or disease state, i.e., arresting its progression;
(iii) alleviating a disease or disease state, i.e., causing its regression; and
(iv) reducing any direct or indirect pathological consequences of a disease or disease state.

The term "therapeutically effective amount" refers to an amount of the compound of the present disclosure for (i) treating or preventing a specific disease, condition, or disorder, (ii) relieving, ameliorating, or eliminating one or more symptoms of a specific disease, condition, or disorder, or (iii) preventing or delaying the onset of one or more symptoms of the specific disease, condition, or disorder described herein. The amount of the compound of the present disclosure that constitutes a "therapeutically effective amount" may vary depending on factors such as the compound and its ability to elicit a desired response in an individual, the disease state and its severity, the mode of administration, and the age, sex, and body weight of the mammal to be treated.

The term "administer", "administration", or "administering" refers to physically introducing a therapeutic agent to a subject using any one of a variety of methods and delivery systems known to those skilled in the art.

Routes of administration for antibody-drug conjugates (e.g., anti-HER2 antibody-drug conjugates) include intravenous, intramuscular, intraperitoneal, spinal, or other parenteral routes of administration. As used herein, the term "parenteral administration" refers to modes of administration other than enteral administration, typically performed by injection, including, but not limited to, intravenous, intramuscular, intra-arterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural, and intrasternal injection and infusion, as well as *in vivo* electroporation. The administration may also be performed, for example, once, multiple times, and/or over one or more extended periods of time.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" refers to a salt of a compound (e.g., the antibody-drug conjugate of the present disclosure) that exhibits safety and efficacy when used in mammals and possesses the desired biological activity; for example, such a salt may be a metal salt, an ammonium salt, a salt formed with an organic base, a salt formed with an inorganic acid, a salt formed with an organic acid, a salt formed with a basic or acidic amino acid, or the like.

The term "excipient" refers to any ingredient other than the active ingredient (e.g., the antibody-drug conjugate of the present disclosure). The selection of the excipient will largely depend on factors such as the particular mode of administration, the effect of the excipient on solubility and stability, and the nature of the dosage form.

The term "solvate" refers to a substance formed by association of a compound with a solvent molecule.

In the present disclosure, "HER2-positive" intestinal cancer is not particularly limited as long as it is recognized by those skilled in the art as intestinal cancer with HER2 overexpression, and preferred examples include intestinal cancer determined to have HER2 expression of 3+ by immunohistochemistry (IHC) (i.e., determined as IHC 3+ by HER2 testing using IHC), or/and intestinal cancer determined to have HER2 expression of 2+ by IHC and determined to be positive for HER2 expression by in situ hybridization (ISH) (i.e., determined as IHC 2+ by HER2 testing using IHC and determined as ISH+ by HER2 testing using ISH). It should be noted that the in situ hybridization of the present disclosure includes, but is not limited to, fluorescence in situ hybridization (FISH) and dual-color in situ hybridization (DISH).

Herein, the terms "subject", "patient", and "entity" can be used interchangeably. The "subject", "patient", or "entity" includes any human or non-human animal. The term "non-human animal" includes, but is not limited to, vertebrates such as non-human primates, sheep, dogs, and rodents such as mice, rats, and guinea pigs. In some embodiments, the subject, patient, or entity is a mammal. In some embodiments, the subject, patient, or entity is a mouse. In some embodiments, the subject, patient, or entity is a human.

The term "pharmaceutical composition" refers to a mixture composed of one or more active ingredients (e.g., the anti-HER2 antibody-drug conjugate of the present disclosure) and pharmaceutically acceptable excipients. The pharmaceutical composition is intended to facilitate the administration of the active ingredient(s) to a subject. Herein, the terms "pharmaceutical composition" and "formulation" have the same meaning and can be used interchangeably. The word "comprise" and variations thereof such as "comprises" or "comprising" should be understood in an openended and non-exclusive sense, i.e., "including but not limited to".

Herein, unless otherwise specified clearly in the context, singular terms encompass plural referents, and vice versa. As used herein, "about" means being within an acceptable error range determined by those of ordinary skill in the art for a specific value, which will depend in part on how the value is measured or determined, i.e., the limitation by the measurement system. For example, "about" may mean being within 1× or more than 1× standard deviation, as practiced in the art. Alternatively, "about" may mean a range of up to ±5%, for example, fluctuating within a specific numerical range given ±2%, ±1%, or ±0.5%. Where a specific value is given in the present disclosure or in the claims, unless otherwise stated, "about" should be considered to mean being within an acceptable error range for that specific value. Herein, unless otherwise stated, all values for drug doses, timing, step parameters, or conditions are modified by "about" by default.

All patents, patent applications, and other identified publications are explicitly incorporated herein by reference for the purpose of description and disclosure. These publications are provided solely because they were disclosed prior to the filing date of the present disclosure. All statements as to the dates of these documents or descriptions as to the content of these documents are based on the information available to the applicant and do not constitute any admission as to the correctness of the dates or the content of these documents. Moreover, in any country or region, any reference to these publications herein shall not be construed as an admission that the publications form part of the commonly recognized knowledge in the art.

The present disclosure further provides the following specific embodiments, which, however, are not intended to limit the protection scope of the present disclosure:
Embodiment 1. A method for treating intestinal cancer in a subject, comprising administering to the subject an anti-HER2 antibody-drug conjugate, wherein the anti-HER2 antibody-drug conjugate is formed by linking a drug-linker having a structure represented by formula Ia below 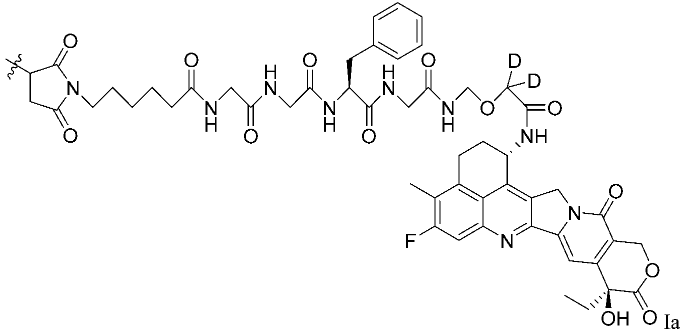
   to an HER2-targeting antigen-binding construct, wherein
   position 3 of -(succinimid-3-yl-N)- in formula Ia is linked to the HER2-targeting antigen-binding construct;
   the HER2-targeting antigen-binding construct comprises a first antigen-binding fragment and a second antigen-binding fragment, wherein the first antigen-binding fragment comprises: an HCDR1 comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6, and the second antigen-binding fragment comprises: an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.
Embodiment 2. The method according to embodiment 1, wherein an average number of linked drug-linkers per HER2-targeting antigen-binding construct is 4 to 7.
Embodiment 3. The method according to embodiment 2, wherein the average number of linked drug-linkers per HER2-targeting antigen-binding construct is 5 to 6.
Embodiment 4. The method according to embodiment 3, wherein the average number of linked drug-linkers per HER2-targeting antigen-binding construct is 5.5 to 6.
Embodiment 5. The method according to any one of embodiments 1-4, wherein
   (i) the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7;
   (ii) the first antigen-binding fragment comprises a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8;
   (iii) the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8;
   (iv) the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15;
   (v) the second antigen-binding fragment comprises a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16;
   (vi) the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16; or
   (vii) the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8; and, the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16.
Embodiment 6. The method according to any one of embodiments 1-5, wherein
   (i) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17 or 20;
   (ii) the HER2-targeting antigen-binding construct comprises a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18 or 21;
   (iii) the HER2-targeting antigen-binding construct comprises a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19;
   (iv) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17 or 20, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18 or 21, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19;
   (v) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19;
   (vi) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 20, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 21, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19;
   (vii) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 21, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19; or
   (viii) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 20, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19.
Embodiment 7. The method according to any one of embodiments 1-6, wherein the anti-HER2 antibody-drug conjugate is administered in a therapeutically effective amount.
Embodiment 8. The method according to any one of embodiments 1-6, wherein the anti-HER2 antibody-drug conjugate is administered at a dose of 1.5-9 mg/kg, 4.5-7.5 mg/kg, or 6-7.5 mg/kg each time; preferably, the anti-HER2 antibody-drug conjugate is administered at a dose of 6-7.5 mg/kg each time.
Embodiment 9. The method according to embodiment 8, wherein the anti-HER2 antibody-drug conjugate is administered at a dose of 1.5 mg/kg, 3 mg/kg, 4.5 mg/kg, 5 mg/kg, 6 mg/kg, 7.5 mg/kg, or 9 mg/kg each time; preferably, the anti-HER2 antibody-drug conjugate is administered at a dose of 6 mg/kg or 7.5 mg/kg each time.
Embodiment 10. The method according to any one of embodiments 1-9, wherein the anti-HER2 antibody-drug conjugate is administered once every 1 week, every 2 weeks, every 3 weeks, or every 4 weeks; preferably, the anti-HER2 antibody-drug conjugate is administered once every 3 weeks.
Embodiment 11. The method according to any one of embodiments 1-10, wherein the anti-HER2 antibody-drug conjugate is formulated into a formulation for intravenous injection or infusion.
Embodiment 12. The method according to any one of embodiments 1-11, wherein the anti-HER2 antibody-drug conjugate is administered by intravenous infusion.
Embodiment 13. The method according to any one of embodiments 1-12, wherein the intestinal cancer is locally advanced intestinal cancer.
Embodiment 14. The method according to any one of embodiments 1-12, wherein the intestinal cancer is intestinal cancer with distant metastasis.
Embodiment 15. The method according to any one of embodiments 1-14, wherein the intestinal cancer is unresectable intestinal cancer.
Embodiment 16. The method according to any one of embodiments 1-15, wherein the intestinal cancer is HER2-expressing intestinal cancer.
Embodiment 17. The method according to any one of embodiments 1-16, wherein the intestinal cancer is HER2-positive intestinal cancer.
Embodiment 18. The method according to embodiment 17, wherein the HER2-positive intestinal cancer is intestinal cancer determined to have HER2 expression of 3+ by IHC.
Embodiment 19. The method according to embodiment 17, wherein the HER2-positive intestinal cancer is intestinal cancer determined to have HER2 expression of 2+ by IHC and determined to be positive for HER2 expression by ISH.
Embodiment 20. The method according to any one of embodiments 1-19, wherein the subject with intestinal cancer has previously received at least second-line therapy for treating intestinal cancer.
Embodiment 21. The method according to any one of embodiments 1-20, wherein the subject with intestinal cancer has previously received chemotherapy for treating intestinal cancer.
Embodiment 22. The method according to any one of embodiments 1-21, wherein the subject with intestinal cancer has previously received standard therapy for treating intestinal cancer.
Embodiment 23. The method according to embodiment 22, wherein the standard therapy comprises administering to the subject fluorouracil or a derivative thereof, oxaliplatin, and/or irinotecan.
Embodiment 24. The method according to any one of embodiments 1-23, wherein the subject with intestinal cancer has previously received adjuvant therapy for treating intestinal cancer.
Embodiment 25. The method according to embodiment 24, wherein the adjuvant therapy comprises administering oxaliplatin to the subject.
Embodiment 26. The method according to any one of embodiments 1-25, wherein the subject with intestinal cancer has previously received local radiation therapy for treating intestinal cancer.
Embodiment 27. The method according to any one of embodiments 1-26, wherein the intestinal cancer is colon cancer, rectal cancer, or colorectal cancer.
Embodiment 28. Use of an anti-HER2 antibody-drug conjugate for preparing a medicament for treating intestinal cancer in a subject, wherein the anti-HER2 antibody-drug conjugate is formed by linking a drug-linker having a structure represented by formula Ia below 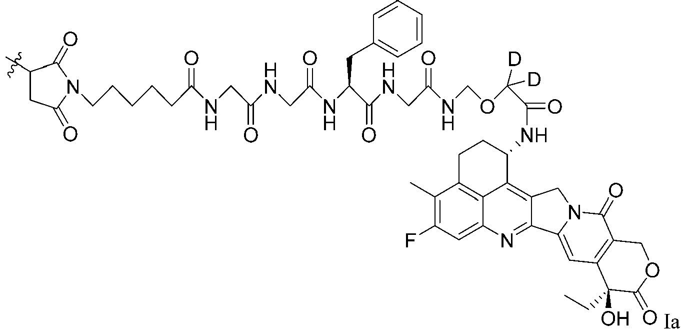
   to an HER2-targeting antigen-binding construct, wherein
   position 3 of -(succinimid-3-yl-N)- in formula Ia is linked to the HER2-targeting antigen-binding construct;
   the HER2-targeting antigen-binding construct comprises a first antigen-binding fragment and a second antigen-binding fragment, wherein the first antigen-binding fragment comprises: an HCDR1 comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6, and the second antigen-binding fragment comprises: an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.
Embodiment 29. The use according to embodiment 28, wherein an average number of linked drug-linkers per HER2-targeting antigen-binding construct is 4 to 7.
Embodiment 30. The use according to embodiment 29, wherein the average number of linked drug-linkers per HER2-targeting antigen-binding construct is 5 to 6.
Embodiment 31. The use according to embodiment 30, wherein the average number of linked drug-linkers per HER2-targeting antigen-binding construct is 5.5 to 6.
Embodiment 32. The use according to any one of embodiments 28-31, wherein
   (i) the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7;
   (ii) the first antigen-binding fragment comprises a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8;
   (iii) the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8;
   (iv) the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15;
   (v) the second antigen-binding fragment comprises a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16;
   (vi) the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16; or
   (vii) the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8; and, the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16.
Embodiment 33. The use according to any one of embodiments 28-32, wherein
   (i) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17 or 20;
   (ii) the HER2-targeting antigen-binding construct comprises a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18 or 21;
   (iii) the HER2-targeting antigen-binding construct comprises a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19;
   (iv) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17 or 20, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18 or 21, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19;
   (v) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19;
   (vi) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 20, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 21, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19;
   (vii) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 21, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19; or
   (viii) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 20, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19.
Embodiment 34. The use according to any one of embodiments 28-33, wherein the medicament comprises a therapeutically effective amount of the anti-HER2 antibody-drug conjugate.
Embodiment 35. The use according to any one of embodiments 28-33, wherein the anti-HER2 antibody-drug conjugate is administered at a dose of 1.5-9 mg/kg, 4.5-7.5 mg/kg, or 6-7.5 mg/kg each time; preferably, the anti-HER2 antibody-drug conjugate is administered at a dose of 6-7.5 mg/kg each time.
Embodiment 36. The use according to embodiment 35, wherein the anti-HER2 antibody-drug conjugate is administered at a dose of 1.5 mg/kg, 3 mg/kg, 4.5 mg/kg, 5 mg/kg, 6 mg/kg, 7.5 mg/kg, or 9 mg/kg each time; preferably, the anti-HER2 antibody-drug conjugate is administered at a dose of 6 mg/kg or 7.5 mg/kg each time.
Embodiment 37. The use according to any one of embodiments 28-36, wherein the anti-HER2 antibody-drug conjugate is administered once every 1 week, every 2 weeks, every 3 weeks, or every 4 weeks; preferably, the anti-HER2 antibody-drug conjugate is administered once every 3 weeks.
Embodiment 38. The use according to any one of embodiments 28-37, wherein the anti-HER2 antibody-drug conjugate is formulated into a formulation for intravenous injection or infusion.
Embodiment 39. The use according to any one of embodiments 28-38, wherein the anti-HER2 antibody-drug conjugate is administered by intravenous infusion.
Embodiment 40. The use according to any one of embodiments 28-39, wherein the intestinal cancer is locally advanced intestinal cancer.
Embodiment 41. The use according to any one of embodiments 28-39, wherein the intestinal cancer is intestinal cancer with distant metastasis.
Embodiment 42. The use according to any one of embodiments 28-41, wherein the intestinal cancer is unresectable intestinal cancer.
Embodiment 43. The use according to any one of embodiments 28-42, wherein the intestinal cancer is HER2-expressing intestinal cancer.
Embodiment 44. The use according to any one of embodiments 28-43, wherein the intestinal cancer is HER2-positive intestinal cancer.
Embodiment 45. The use according to embodiment 44, wherein the HER2-positive intestinal cancer is intestinal cancer determined to have HER2 expression of 3+ by IHC.
Embodiment 46. The use according to embodiment 44, wherein the HER2-positive intestinal cancer is intestinal cancer determined to have HER2 expression of 2+ by IHC and determined to be positive for HER2 expression by ISH.
Embodiment 47. The use according to any one of embodiments 28-46, wherein the subject with intestinal cancer has previously received at least second-line therapy for treating intestinal cancer.
Embodiment 48. The use according to any one of embodiments 28-47, wherein the subject with intestinal cancer has previously received chemotherapy for treating intestinal cancer.
Embodiment 49. The use according to any one of embodiments 28-48, wherein the subject with intestinal cancer has previously received standard therapy for treating intestinal cancer.
Embodiment 50. The use according to embodiment 49, wherein the standard therapy comprises administering to the subject fluorouracil or a derivative thereof, oxaliplatin, and/or irinotecan.
Embodiment 51. The use according to any one of embodiments 28-50, wherein the subject with intestinal cancer has previously received adjuvant therapy for treating intestinal cancer.
Embodiment 52. The use according to embodiment 51, wherein the adjuvant therapy comprises administering oxaliplatin to the subject.
Embodiment 53. The use according to any one of embodiments 28-52, wherein the subject with intestinal cancer has previously received local radiation therapy for treating intestinal cancer.
Embodiment 54. The use according to any one of embodiments 28-53, wherein the intestinal cancer is colon cancer, rectal cancer, or colorectal cancer.

### BRIEF DESCRIPTION OF THE DRAWINGS

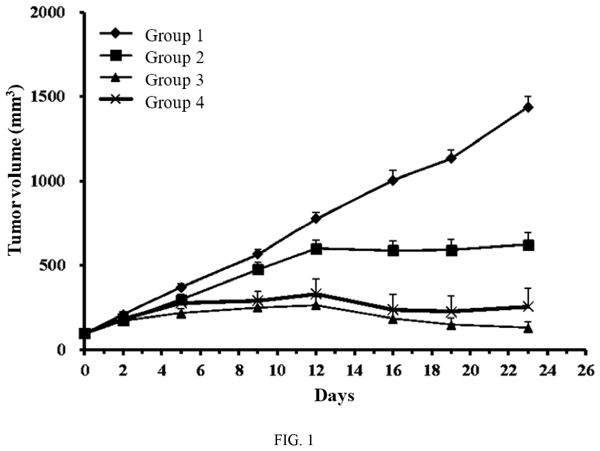

FIG. 1 shows the tumor volume growth curves of COLO-205 tumor-bearing mice in a vehicle control group, an ADC1 (3 mg/kg) administration group, an ADC1 (10 mg/kg) administration group, and a DS-8201a (10 mg/kg) administration group.

### Example

For clarity, the present disclosure is further described with the following examples, which are, however, not intended to limit the scope of the present disclosure.

The content of the patent application WO2022033578 or CN115702008A is incorporated herein by reference in its entirety. In the following examples, the anti-HER2 antibody-drug conjugate is prepared according to the preparation method described in WO2022033578 to obtain the anti-HER2 antibody-drug conjugate represented by the formula below (hereinafter referred to as ADC1): 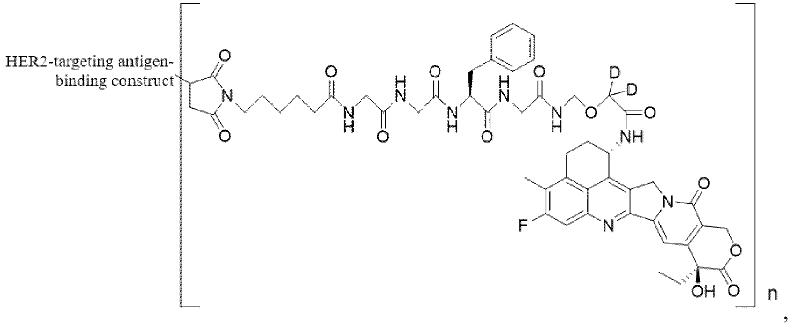 wherein the drug-linker is linked to the HER2-targeting antigen-binding construct via a thioether bond, and n is 5 to 6. Briefly, the nucleic acid sequences encoding the three polypeptide chains of the HER2-targeting antigen-binding construct (with amino acid sequences set forth in SEQ ID NOs: 17, 18, and 19, respectively) are each cloned into a pcDNA3.1 expression vector, and the vectors were co-transfected into FUT8-knockout CHO-S cells for expression. The HER2-targeting antigen-binding construct is then prepared after purification via Protein A. The HER2-targeting antigen-binding construct is treated with tris(2-carboxyethyl)phosphine hydrochloride and subsequently reacted with a linker-payload selected from the structure represented by the formula below to finally obtain ADC1:

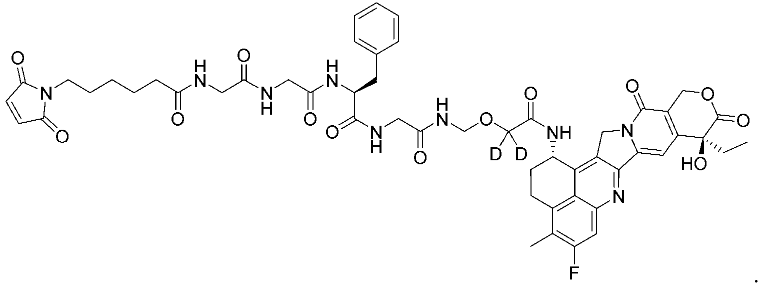

### Example 1: Anti-Tumor Effect of Anti-HER2 Antibody-Drug Conjugate in COLO-205 Mouse Tumor Model

BALB/c Nude mice (from GemPharmatech) were inoculated subcutaneously on the right dorsal side with COLO-205 (from Chinese Academy of Sciences, Shanghai Institutes for Biological Sciences) human colon cancer tumor cells in an amount of 5 × 10⁶ cells/mouse. When the *in vivo* tumor volume grew to about 100 mm³ on average, the tumor-bearing mice were randomly divided into 4 groups of 6. The day of grouping was defined as day 0, and administration was performed by intravenous injection according to the regimen in Table 2 from day 0. DS-8201a (trastuzumab deruxtecan) from Daiichi Sankyo was used as a positive control, and a 5% glucose solution was used as a vehicle control. After the start of administration, the body weight and tumor size of the mice were measured twice a week, and the tumor growth inhibition (TGI) was calculated.$Tumor volume \left({mm}^{3}\right) = 0.5 \times \left(long diameter of tumor\times short diameter of {tumor}^{2}\right) ;$$TGI \left(%\right) = \left(1-T/C\right) \times 100 % ;$$T / C = TRTV / CRTV \times 100 % ,$ wherein T_{RTV} represents the mean relative tumor volume (RTV) for treatment group; C_{RTV} represents mean RTV for vehicle control group; RTV = Vₜ/V₀, where V₀ represents the tumor volume of the animal upon grouping, and Vₜ represents the tumor volume of the animal after treatment.

**Table 2. Administration regimen**

| Group | Administration | Dose (mg/kg) | Dose volume (µL/g) | Time of administration |
|---|---|---|---|---|
| 1 | Vehicle control | - | 10 | Twice a week |
| 2 | ADC1 | 3 | 10 | Twice a week |
| 3 | ADC1 | 10 | 10 | Twice a week |
| 4 | DS-8201a | 10 | 10 | Twice a week |

The results are shown in Table 3 and FIG. 1. The anti-tumor activity of ADC1 was superior to that of DS-8201a. It can be seen that the anti-HER2 antibody-drug conjugate of the present disclosure may have good anti-tumor activity in animals with colon cancer.

**Table 3. Anti-tumor effect of anti-HER2 antibody-drug conjugate in COLO-205 mouse tumor model**

| Group | Administration | Dose (mg/kg) | Mean tumor volume on day 23 (mm³) | TGI on day 23 |
|---|---|---|---|---|
| 1 | Vehicle control | - | 1439 | - |
| 2 | ADC1 | 3 | 623 | 56.71% |
| 3 | ADC1 | 10 | 132 | 90.83% |
| 4 | DS-8201a | 10 | 257 | 82.15% |

### Example 2: Phase I Clinical Trial for HER2-Expressing Malignant Tumor

### 1. Study objectives:

### 1.1 Primary objective:

To evaluate the tolerability of ADC1 in subjects with advanced malignant tumors.

### 1.2 Secondary objectives:

To evaluate the pharmacokinetics (PK), immunogenicity, safety, and preliminary efficacy of ADC1 in subjects with advanced malignant tumors;

To evaluate potential biomarkers related to the mechanism of action of ADC1 in subjects with advanced malignant tumors.

### 2. Inclusion criteria

Subjects who meet all of the following inclusion criteria can be enrolled in this trial:
(1) Subjects voluntarily participated in this study and signed the informed consent form;
(2) Age: 18-75 years old (at the time of signing the informed consent form); ECOG score: ≤ 1; expected survival time of more than 3 months;
(3) Dose escalation phase: subjects with cytologically/histologically confirmed advanced malignant tumors (including HER2-positive colorectal cancer), with priority given to those with HER2 expression or amplification or mutation;
   Extended study phase: subjects with unresectable locally advanced colorectal cancer or colorectal cancer with distant metastasis whose tumors were confirmed to be HER2-positive by pathological examination (HER2 IHC 3+, or HER2 IHC 2+ and ISH-positive);
(4) Subjects with malignant tumors who had failed standard therapy or lacked efficacy therapy (including failure of first-line therapy, second-line therapy, third-line therapy, and a later line of therapy, e.g., failure of prior treatment with anti-HER2 ADCs, trastuzumab, pertuzumab, pyrotinib, lapatinib, regorafenib, fruquintinib, bevacizumab, and cetuximab); specific requirements for expansion cohorts are as follows:
   For colorectal cancer, it was required that the patient had previously received ≥ second-line standard chemotherapy, and had experienced disease progression or intolerance during standard therapy or within 3 months after the end of treatment. Standard therapy regimens must include fluorouracil or a derivative thereof, oxaliplatin, irinotecan, etc.; patients who had received adjuvant therapy with oxaliplatin should have experienced disease progression during adjuvant therapy or within 6 months after the completion of adjuvant therapy; the intolerance was defined as grade IV hematological toxicity (a reduction in platelet count of grade III or higher) and grade III or higher non-hematological toxicity;
(5) Having at least one evaluable lesion (not necessarily measurable, but only satisfying the requirement for assessing disease state) as confirmed according to RECIST 1.1 criteria in the dose-escalation phase, and having at least one measurable lesion as confirmed according to RECIST 1.1 criteria in the cohort-expansion phase;
(6) Good function of major organs, meeting the criteria for blood routine examination, biochemical examination, coagulation function examination, and cardiac color ultrasound assessment:
(7) Female subjects of childbearing age should agree to take necessary contraceptive measures (such as intrauterine devices, contraceptives, or condoms) during the study and for 6 months after the study; the serum pregnancy test results should be negative within 7 days before enrollment, and the subjects must be in the non-lactation period; male subjects should agree to take necessary contraceptive measures during the study and for 6 months after the study.

### 3. Test drug

ADC1 for injection (specification: 100 mg/vial), developed and provided by Chia Tai Tianqing Pharmaceutical Group Nanjing Shunxin Pharmaceutical Co., Ltd.

### 4. Treatment regimen

### 4.1 Dose escalation phase:

Dose escalation was performed in 6 dose groups, and the dose levels were 1.5 mg/kg, 3 mg/kg, 4.5 mg/kg, 6 mg/kg, 7.5 mg/kg, and 9 mg/kg, respectively. One treatment cycle consisted of 3 weeks (21 days), and ADC1 was administered once per treatment cycle until disease progression or ineligibility as determined by the investigator.

### 4.2 Extended study phase:

1 to 2 appropriate dose groups (including 6 mg/kg and 7.5 mg/kg) were selected in the dose-escalation phase. One treatment cycle consisted of 3 weeks (21 days), and ADC1 was administered once per treatment cycle until disease progression or ineligibility as determined by the investigator.

The treatment regimen could be adjusted according to the severity of the disease, the response of the disease, any treatment-related toxicity, and the age and health status of a patient.

### 5. Evaluation criteria

Efficacy evaluation: the disease status was determined using the RECIST 1.1 criteria.

Safety evaluation: the severity of adverse events was determined using the NCI-CTC AE 5.0 criteria.

### 6. Endpoint indicators

### 6.1 Primary endpoints:

Dose-limiting toxicity (DLT), maximum tolerated dose (MTD), and recommended phase II dose (RP2D); Incidence and severity of adverse event (AE);

### 6.2 Secondary endpoints:

Immunogenicity (e.g., ADA incidence);
Pharmacokinetic parameters;
Objective response rate (ORR), disease control rate (DCR), duration of response (DOR), progression-free survival (PFS), and overall survival (OS).

### 7. Results

By the date of clinical data collection, a total of 24 patients with stage IV colorectal cancer who were enrolled in the 7.5 mg/kg dose group (covering patients with primary lesion sites of rectum, sigmoid colon, and colon) had reached an evaluable level, with ORR of about 29% (7/24) and DCR of 87.5% (21/24). Particularly, in IHC 3+ HER2-positive patients (a total of 20 patients), a better effect (ORR of up to 35%) was observed. In the above dose groups, the median time to response for the ADC1 therapy reached 2.66 months, the median progression-free survival (mPFS) reached 6.7 months (95% CI), and the incidence of interstitial lung diseases was 0. Of the 24 evaluable patients, 66.6% (16/24) had previously received anti-HER2 therapy, including 5 patients who had previously received anti-HER2 ADCs.

The anti-HER2 antibody-drug conjugate of the present disclosure can show good therapeutic efficacy and safety in patients with HER2-positive intestinal cancer, particularly colorectal cancer.

### Example 3: Phase III Clinical Trial for HER2 IHC 3+ Advanced Colorectal Cancer

### 1. Study objectives:

### 1.1 Primary objective:

To compare the efficacy of ADC1 versus standard therapy (trifluridine and tipiracil hydrochloride tablets) in patients with HER2 IHC 3+ advanced colorectal cancer who had previously received treatment with oxaliplatin, 5-fluorouracil, and irinotecan, as measured by the progression-free survival (PFS) of the disease as assessed by the independent review committee (IRC).

### 1.2 Key secondary objectives:

To compare the efficacy of ADC1 versus standard therapy (trifluridine and tipiracil hydrochloride tablets) as measured by the overall survival (OS).

### 1.3 Secondary objectives:

To compare the efficacy of ADC1 versus standard therapy (trifluridine and tipiracil hydrochloride tablets) as measured by PFS, ORR, DCR, DOR and time to response (TTR) as assessed by the investigator;

To evaluate the safety and tolerability of ADC1 versus standard therapy (trifluridine and tipiracil hydrochloride tablets).

### 2. Inclusion criteria

Subjects who meet all of the following inclusion criteria can be enrolled in this trial:
(1) Subjects voluntarily participated in this study, signed the informed consent form, and demonstrated good compliance;
(2) Age: 18 years ≤ age ≤ 75 years (calculated based on the date of signing the informed consent form);
(3) Subjects with histologically or cytologically confirmed advanced colorectal cancer;
(4) Subjects confirmed to be HER2 IHC 3+ in tumor tissue;
(5) Subjects who must be able to provide a sufficient number of qualified tumor specimens (fresh or archival samples collected within 3 years from primary or metastatic specimens) for central laboratory HER2 status testing;
(6) Subjects with advanced colorectal cancer who had previously failed at least second-line standard therapy (defined as disease progression or intolerance to toxicity during the last standard treatment or within 3 months after the last treatment):
   ● Subjects who had failed prior treatment with oxaliplatin, irinotecan, and 5-fluorouracil (subjects known to have dMMR/MSI-H also had failed an anti-PD-1/PD-L1 antibody);
   ● Subjects whose initial systemic treatment included oxaliplatin, irinotecan, or 5-fluorouracil were counted as having received second-line systemic therapy;
   ● Subjects who had experienced a relapse ≤ 6 months after the last adjuvant therapy were counted as having received first-line systemic therapy;
   ● Subjects with indications for the use of trifluridine and tipiracil hydrochloride tablets as standard therapy after randomization were allowed to have previously received treatment with VEGFR-targeting small-molecule inhibitors, such as regorafenib or fruquintinib;
   ● Intolerance to toxicity of prior treatment was defined as the occurrence of grade 3 or higher AEs that led to the termination of the treatment or the subject requested to terminate the treatment actively due to intolerance despite symptomatic and supportive treatment;
(7) ECOG score of 0-1;
(8) Expected survival greater than 12 weeks;
(9) Having at least one measurable lesion as confirmed according to RECIST 1.1 criteria;
(10) Subjects who met the criteria of blood routine examination, biochemical examination, urine routine examination, blood coagulation function examination and thyroid stimulating hormone examination and were in a state of electrolyte balance;
(11) Female subjects of childbearing age should agree to take effective contraceptive measures during the study and for 6 months after the study; serum or urine pregnancy test results should be negative within 7 days before enrollment; male subjects should agree to take effective contraceptive measures during the study and for 6 months after the study.

### 3. Test drug

ADC1 for injection (specification: 100 mg/vial), developed and provided by Chia Tai Tianqing Pharmaceutical Group Nanjing Shunxin Pharmaceutical Co., Ltd.

ADC1 placebo for injection, provided by Chia Tai Tianqing Pharmaceutical Group Nanjing Shunxin Pharmaceutical Co., Ltd.

Trifluridine and tipiracil hydrochloride tablets (specification: 15 mg/tablet or 20 mg/tablet), provided by Chia Tai Tianqing Pharmaceutical Group Nanjing Shunxin Pharmaceutical Co., Ltd.

Trifluridine and tipiracil hydrochloride placebo, provided by Chia Tai Tianqing Pharmaceutical Group Nanjing Shunxin Pharmaceutical Co., Ltd.

### 4. Experimental design and treatment regimen

Subjects meeting the enrollment criteria were randomly assigned to the test group or the control group in a 1:1 ratio.

### 4.1 Grouping

Test groups: ADC1, 7.5 mg/kg, intravenous drip, administered on day 1 of each treatment cycle; one treatment cycle consisted of 3 weeks; trifluridine and tipiracil hydrochloride placebo, administered orally twice daily on days 1-5 and days 8-12; one treatment cycle consisted of 4 weeks.

Control group: trifluridine and tipiracil hydrochloride tablets, 35 mg/m²/dose (the maximum single dose is 80 mg), administered orally twice daily on days 1-5 and days 8-12; one treatment cycle consisted of 4 weeks; ADC1 placebo, administered by intravenous drip on day 1 of each treatment cycle; one treatment cycle consisted of 3 weeks.

The treatment regimen could be adjusted according to the severity of the disease, the response of the disease, any treatment-related toxicity, and the age and health status of a patient.

### 5. Evaluation criteria

Efficacy evaluation: the disease status was determined using the RECIST 1.1 criteria.

Safety evaluation: the safety evaluation variables include physical examination, vital signs, 12-lead electrocardiogram, laboratory examination, specialized examination, all AEs (including SAEs), and the like.

### 6. Endpoint indicators

### 6.1 Primary endpoints:

PFS assessed by IRC;

### 6.2 Key secondary endpoints:

OS;

### 6.3 Other secondary endpoints:

PFS, ORR, DOR, DCR, TTR, and safety as assessed by the investigator.

After administration of the anti-HER2 antibody-drug conjugate of the present disclosure, patients with colorectal cancer may be expected to achieve clinical benefits, including expected efficacy and safety.

## Claims

1. An anti-HER2 antibody-drug conjugate for use in treating intestinal cancer in a subject, wherein the anti-HER2 antibody-drug conjugate is formed by linking a drug-linker having a structure represented by formula Ia below 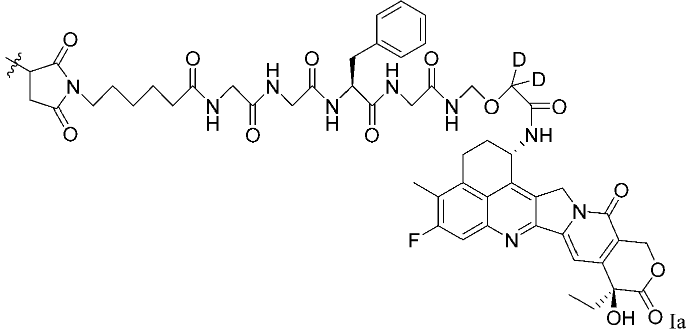
to an HER2-targeting antigen-binding construct, wherein
position 3 of -(succinimid-3-yl-N)- in formula Ia is linked to the HER2-targeting antigen-binding construct; the HER2-targeting antigen-binding construct comprises a first antigen-binding fragment and a second antigen-binding fragment,
wherein the first antigen-binding fragment comprises: an HCDR1 comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6, and the second antigen-binding fragment comprises: an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.

2. The anti-HER2 antibody-drug conjugate for use in treating intestinal cancer in the subject according to claim 1, wherein an average number of linked drug-linkers per HER2-targeting antigen-binding construct is 4 to 7, 5 to 6, or 5.5 to 6.

3. The anti-HER2 antibody-drug conjugate for use in treating intestinal cancer in the subject according to claim 1 or 2, wherein
(i) the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7;
(ii) the first antigen-binding fragment comprises a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8;
(iii) the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8;
(iv) the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15;
(v) the second antigen-binding fragment comprises a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16;
(vi) the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16; or
(vii) the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8; and the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16.

4. The anti-HER2 antibody-drug conjugate for use in treating intestinal cancer in the subject according to any one of claims 1-3, wherein
(i) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17 or 20;
(ii) the HER2-targeting antigen-binding construct comprises a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18 or 21;
(iii) the HER2-targeting antigen-binding construct comprises a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19;
(iv) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17 or 20, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18 or 21, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19;
(v) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19;
(vi) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 20, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 21, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19;
(vii) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 21, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19; or
(viii) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 20, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19.

5. The anti-HER2 antibody-drug conjugate for use in treating intestinal cancer in the subject according to any one of claims 1-4, wherein the anti-HER2 antibody-drug conjugate is administered at a dose of 1.5-9 mg/kg, 4.5-7.5 mg/kg, or 6-7.5 mg/kg each time; preferably, the anti-HER2 antibody-drug conjugate is administered at a dose of 6-7.5 mg/kg each time, or the anti-HER2 antibody-drug conjugate is administered at a dose of 1.5 mg/kg, 3 mg/kg, 4.5 mg/kg, 5 mg/kg, 6 mg/kg, 7.5 mg/kg, or 9 mg/kg each time.

6. The anti-HER2 antibody-drug conjugate for use in treating intestinal cancer in the subject according to any one of claims 1-5, wherein the anti-HER2 antibody-drug conjugate is administered once every 1 week, every 2 weeks, every 3 weeks, or every 4 weeks; preferably, the anti-HER2 antibody-drug conjugate is administered once every 3 weeks.

7. The anti-HER2 antibody-drug conjugate for use in treating intestinal cancer in the subject according to any one of claims 1-6, wherein the anti-HER2 antibody-drug conjugate is formulated into a formulation for intravenous injection or infusion; preferably, the anti-HER2 antibody-drug conjugate is administered by intravenous infusion.

8. The anti-HER2 antibody-drug conjugate for use in treating intestinal cancer in the subject according to any one of claims 1-7, wherein the intestinal cancer is locally advanced intestinal cancer or intestinal cancer with distant metastasis.

9. The anti-HER2 antibody-drug conjugate for use in treating intestinal cancer in the subject according to any one of claims 1-8, wherein the intestinal cancer is unresectable intestinal cancer;
the intestinal cancer is HER2-expressing intestinal cancer; and/or
the intestinal cancer is HER2-positive intestinal cancer.

10. The anti-HER2 antibody-drug conjugate for use in treating intestinal cancer in the subject according to claim 9,
wherein the HER2-positive intestinal cancer is intestinal cancer determined to have HER2 expression of 3+ by IHC; or
the HER2-positive intestinal cancer is intestinal cancer determined to have HER2 expression of 2+ by IHC and determined to be positive for HER2 expression by ISH.

11. The anti-HER2 antibody-drug conjugate for use in treating intestinal cancer in the subject according to any one of claims 1-10, wherein the subject with intestinal cancer has previously received at least second-line therapy for treating intestinal cancer:
the subject with intestinal cancer has previously received chemotherapy for treating intestinal cancer:
the subject with intestinal cancer has previously received standard therapy for treating intestinal cancer;
the subject with intestinal cancer has previously received adjuvant therapy for treating intestinal cancer; and/or
the subject with intestinal cancer has previously received local radiation therapy for treating intestinal cancer.

12. The anti-HER2 antibody-drug conjugate for use in treating intestinal cancer in the subject according to claim 11, wherein the standard therapy comprises administering to the subject fluorouracil or a derivative thereof, oxaliplatin, and/or irinotecan; or
the adjuvant therapy comprises administering oxaliplatin to the subject.

13. The anti-HER2 antibody-drug conjugate for use in treating intestinal cancer in the subject according to any one of claims 1-12, wherein the subject is a subject with intestinal cancer who has failed prior treatment with an HER2-targeted drug or prior treatment with a VEGFR-targeted drug; preferably, the subject is a subject with intestinal cancer who has failed prior treatment with one or more of anti-HER2 ADCs, trastuzumab, pertuzumab, pyrotinib, lapatinib, regorafenib, fruquintinib, bevacizumab, and cetuximab.

14. The anti-HER2 antibody-drug conjugate for use in treating intestinal cancer in the subject according to any one of claims 1-13, wherein the intestinal cancer is sigmoid colon cancer, colon cancer, rectal cancer, or colorectal cancer.

15. The anti-HER2 antibody-drug conjugate for use in treating intestinal cancer in the subject according to any one of claims 1-14, wherein the intestinal cancer is stage I, stage II, stage III, or stage IV intestinal cancer according to TNM staging criteria.
